(19) Europäisches Patentamt
European Patent Office
Office européen des brevets

(11) Veröffentlichungsnummer: **0 347 370**
**A2**

# (12) EUROPÄISCHE PATENTANMELDUNG

(21) Anmeldenummer: **89730144.6**

(22) Anmeldetag: **16.06.89**

(51) Int. Cl.4: **C 07 J 71/00**
A 61 K 31/58, C 07 J 31/00,
A 61 K 31/585

(30) Priorität: **16.06.88 DE 3820948**

(43) Veröffentlichungstag der Anmeldung:
**20.12.89 Patentblatt 89/51**

(84) Benannte Vertragsstaaten:
**AT BE CH DE ES FR GB GR IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Scheidges, Cornelius, Dr.**
**Caspar-Theyss-Strasse 19**
**D-1000 Berlin 33 (DE)**

**Ottow, Eckhard, Dr.**
**Sonnenallee 124**
**D-1000 Berlin 44 (DE)**

**Neef, Günter, Dr.**
**Markgraf-Albrecht Str. 4**
**D-1000 Berlin 15 (DE)**

**Beier, Sybille, Dr.**
**Uhlandstrasse 121**
**D-1000 Berlin 31 (DE)**

**Elger, Walter, Dr.**
**Schorlemer Allee 12B**
**D-1000 Berlin 33 (DE)**

## (54) 10beta, 11beta-überbrückte Steroide.

(57) Es werden 10β,11β-überbrückte Steroide der allgemeinen Formel I

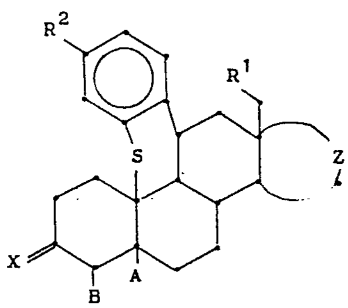

(I),

beschrieben,
worin
$R^1$ für ein H-Atom oder eine Methylgruppe,
$R^2$ für ein H-Atom, einen Cyanidrest, einen Heteroarylrest, eine geradkettige oder verzweigte, gegebenenfalls Doppel- oder Dreifachbindung(en) aufweisende Alkylgruppe mit bis zu 20 C-Atomen, die gegebenenfalls durch eine oder mehrere Oxo-Gruppe(n) substituiert ist, eine $OR^3$-, $SR^3$-, $-OSO_2-R^{11}$-Gruppe mit $R^{11}$ in der Bedeutung einer perfluorierten $C_1$-$C_4$-Alkylgruppe oder $NR^3R^4$-Gruppe mit $R^3$ in der Bedeutung eines H-Atoms oder eines $C_1$-$C_8$-Alkylrestes, $R^4$ in der Bedeutung von $R^3$, eines Cyanid- oder eines $C_1$-$C_{10}$-Acylrestes oder $R^3$ und $R^4$ gemeinsam unter Einschluß von N in der Bedeutung eines 5- oder 6-gliedrigen heterocyclischen Rings, wobei im Ring noch ein weiteres Heteroatom N, O oder S enthalten sein kann,
A und B entweder gemeinsam für eine weitere Bindung zwischen $C_4$ und $C_5$ oder A für eine α-Hydroxygruppe und B für ein H-Atom,
X für eine Keto- oder Oximgruppe und
Z für den Rest eines pentagonalen oder hexagonalen Ringes, der gegebenenfalls substituiert und gegebenenfalls ungesättigt ist,
stehen,
sowie gegebenenfalls deren pharmazeutisch verträgliche Additionssalze mit Säuren.

Die Verbindungen besitzen antigestagene und antiglucocorticoide Eigenschaften.



Bundesdruckerei Berlin

**Beschreibung**

**10β, 11β-überbrückte Steroide**

Die vorliegende Erfindung betrifft 10β,11β-überbrückte Steroide, Verfahren zu ihrer Herstellung, diese Verbindungen enthaltende pharmazeutische Präparate, ihre Verwendung zur Herstellung von Arzneimitteln sowie die dazu benötigten neuen Zwischenprodukte.

Die erfindungsgemäßen 10β,11β-überbrückten Steroide werden durch die allgemeine Formel I beschrieben

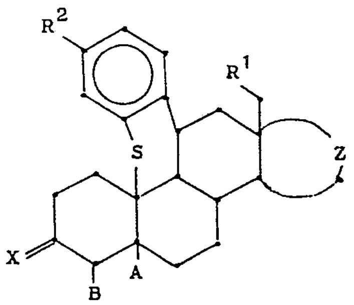

(I),

worin

$R^1$ für ein H-Atom oder eine Methylgruppe,

$R^2$ für ein H-Atom, einen Cyanidrest, einen Heteroarylrest, eine geradkettige oder verzweigte, gegebenenfalls Doppel- oder Dreifachbindung(en) aufweisende Alkylgruppe mit bis zu 20 C-Atomen, die gegebenenfalls durch eine oder mehrere Oxo-Gruppe(n) substituiert ist, eine $OR^3$-, $SR^3$-, $-OSO_2-R^{11}$-Gruppe mit $R^{11}$ in der Bedeutung einer perfluorierten $C_1$-$C_4$-Alkylgruppe oder $NR^3R^4$-Gruppe mit $R^3$ in der Bedeutung eines H-Atoms oder eines $C_1$-$C_8$Alkylrestes, $R^4$ in der Bedeutung von $R^3$, eines Cyanid- oder eines $C_1$-$C_{10}$-Acylrestes oder $R^3$ und $R^4$ gemeinsam unter Einschluß von N in der Bedeutung eines 5- oder 6-gliedrigen heterocyclischen Ringes, wobei im Ring noch ein weiteres Heteroatom N, O oder S enthalten sein kann,

A und B entweder gemeinsam für eine weitere Bindung zwischen C4 und C5 oder A für eine α-Hydroxygruppe und B für ein H-Atom,

X für eine Keto- oder Oximgruppe und

Z für den Rest eines pentagonalen oder hexagonalen Ringes, der gegebenenfalls substituiert und gegebenenfalls ungesättigt ist,

stehen,

sowie gegebenenfalls deren pharmazeutisch verträgliche Additionssalze mit Säuren.

Die Erfindung betrifft insbesondere Verbindungen der allgemeinen Formel I, in denen Z für den Rest eines Ringes der Formel

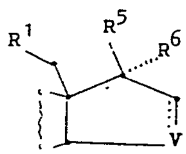

steht, worin

$R^1$ die in Anspruch 1 genannte Bedeutung hat,

die von V ausgehende gestrichelte Linie die etwaige Anwesenheit einer Doppelbindung symbolisiert,

V für einen $CH_2$-, CH-, $CH_2CH_2$- oder $CHCH_2$-Rest steht und

$R^5/R^6$ $-OR^7/-C\equiv C-U$

$-OR^7/-\underset{\overset{\|}{O}}{C}-CH_2-R^8$

$-\underset{\overset{\|}{O}}{C}-CH_2-R^8/-OR^7$

$-\underset{\overset{\|}{O}}{C}-CH_2-R^8/-CH_3$

$-\underset{\overset{\|}{O}}{C}-CH_2-R^8/-H$

$-OR^7/-(CH_2)_m-CH_2-R^9$

$-OR^7/-CH=CH(CH_2)_k-CH_2-R^9$

$-OR^{10}/-H$

$-OR^{10}/-(CH_2)_k-C\equiv C-E$

wobei E Halogen ist,

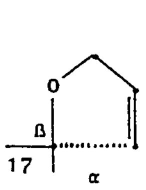 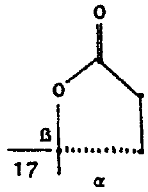

mit $R^7$ in der Bedeutung eines Wasserstoffatoms oder Acylrestes mit 1 bis 4 Kohlenstoffatomen, U in der Bedeutung eines Wasserstoffatoms, einer Alkyl-, Hydroxyalkyl-, Alkoxyalkyl- oder Acyloxyalkylgruppe mit jeweils 1 bis 4 Kohlenstoffatomen im Alkyl- bzw. Acylrest,
$R^8$ in der Bedeutung eines Wasserstoffatoms, einer Hydroxygruppe, einer Alkyl-, O-Alkyl- oder O-Acylgruppe mit jeweils 1 bis 4 Kohlenstoffatomen,
$R^9$ in der Bedeutung eines Wasserstoffatoms, eines Hydroxy- oder Cyanidrestes, einer O-Alkyl- oder O-Acylgruppe mit jeweils 1 bis 4 Kohlenstoffatomen,
$R^{10}$ in der Bedeutung eines Wasserstoffatoms, einer Alkyl- oder Acylgruppe mit jeweils 1 bis 10 Kohlenstoffatomen,
m in der Bedeutung 0, 1, 2 oder 3,
k in der Bedeutung 0, 1 oder 2,
bedeuten.

Die in $R^5$ und $R^6$ bzw. $R^7$, $R^8$, $R^9$, $R^{10}$ und U der allgemeinen Formel I enthaltenen Alkyl-, Alkoxy- sowie Acyloxygruppen sollen jeweils 1 bis 4 Kohlenstoffatome enthalten, wobei die Methyl-, Ethyl-, Propyl-, Isopropyl-, Methoxy-, Ethoxy-, Propoxy-, Isopropoxy-, Formyl-, Acetyl-, Propionyl- und Isopropionylgruppe bevorzugt sind.

Von den Alkenylresten in $R^6$ sind die Propenyl- und Butenylgruppen, die in der E- oder Z-Konfiguration vorliegen können, bevorzugt, das heißt wenn $R^6$ für $-CH=CH-(CH_2)_k-CH_2-R^9$ steht, dann soll k bevorzugt 0 oder 1 bedeuten.

Die neuen Verbindungen der allgemeinen Formel I werden erfindungsgemäß nach dem Verfahren gemäß Anspruch 6 hergestellt.

Zur Herstellung der Ausgangsprodukte der allgemeinen Formel II

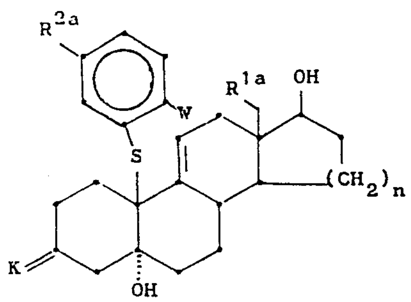 (II),

worin
$R^{1a}$ und $R^{1a}$ die gleiche Bedeutung haben wie $R^1$ bzw. $R^2$ in der allgemeinen Formel I und wobei gegebenenfalls in $R^{2a}$ vorhandene Hydroxy-, Mercapto-, Amino-, Oxo- und/oder terminale Acetylengruppen geschützt sind,
n für die Ziffern 1 oder 2,
K für eine in Form des Ketals oder Thioketals blockierte Ketogruppe und
W für ein Brom- oder Jodatom stehen,
werden ausgehend von den nach den Vorschriften in zum Beispiel Europäische Patentanmeldung Publikationsnummer 0 110 434, DE 34 38 484 oder Europäische Patentanmeldung Publikationsnummer 0 127 864 erhaltenen Epoxiden der allgemeinen Formel IV

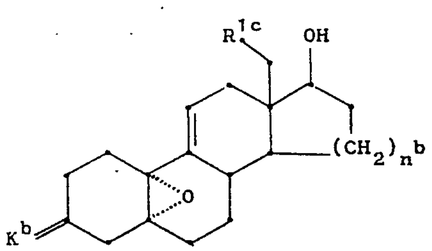 (IV),

worin $R^{1c}$, $K^b$und $n^b$ die gleiche Bedeutung haben wie $R^1$, K und n,

durch nucleophile Addition eines geeingeten Thiophenolats an das Kohlenstoffatom 10 hergestellt.

Dabei können je nach den in den Zwischenprodukten gewünschten Substituenten $R^{2a}$ verschiedene Synthesewege gewählt werden.

Soll der einzuführende Thiophenolatrest in m-Position zum Schwefelatom eine Aminogruppierung ($R^3$, $R^4$ = $CH_3$, $C_2H_5$ sowie die entsprechenden Monoalkylverbindungen) tragen, so kann zunächst 3-Mercaptoanilin nucleophil unter Zuhilfenahme einer starken Base, beispielsweise n-Butyllithium, an das Kohlenstoffatom 10 des Steroidgerüstes addiert und anschließend durch einmalige Acylierung der Aminogruppe und anschließende reduktion der N-Monoacylverbindung die entsprechende N-Monoalkylverbindung bzw. durch zweimalige und sukzessive Acylierung und Reduktion die entsprechende N-Dialkylaminoverbindung hergestellt werden. Vorzugsweise wird das gemischte Anhydrid der Ameisensäure und Essigsäure als Acylierungsmittel verwendet und nach der Reduktion somit die entsprechende Monomethyl- bzw. Dimethylaminoverbindung gewonnen. Weitere anspruchsgemäße Aminoverbindungen können in analoger Weise hergestellt werden.

Bei der Acylierung der Aminogruppe wird die 17-Hydroxygruppe jeweils mitacyliert, anschließend bei der Reduktion bzw. der Aufarbeitung des Reduktionsprodukts aber wieder freigesetzt. Als Reduktionsmittel findet vorzugsweise ein komplexes Hydrid, insbesondere Lithiumaluminiumhydrid, Verwendung.

Ein andere Möglichkeit der Öffnung des Expoxids IV besteht darin, ein Thiophenolat der Formel

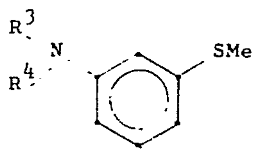

(Me = Li, Na)

nucleophil an das Epoxid IV zu addieren.

In beiden Fällen wird dann durch Halogenierung, vorzugsweise durch Bromierung mit N-Bromsuccinimid, in der o'-Position des Phenylringes ein Halogenatom (Brom oder Jod) eingeführt. Die vorstehend zuerst genannte Reaktionsfolge ist für die Herstellung von 10β-(2-Brom-5-diethylaminophenylthio)-3,3-(2,2-dimethyltrimethylendioxy)-estr-9(11 )-en-5α, 11β-diol im Beispiel 3A bis 3F aufgezeigt.

Bei einer weiteren Variante des erfindungsgemäßen Verfahrens wird das Epoxid der Formel IV direkt mit einem 2-Brom- oder 2-Jod-thiophenolat umgesetzt, das in der 5-Stellung bereits den im Zwischenprodukt der Formel II gewünschten Substituenten $R^{2a}$- trägt oder einen Vorläufer von diesem beispielsweise mit 2-Brom-5-methoxy-natriumthiophenolat.

Gegebenenfalls nach Schutz der am Phenylring vorhandenen funktionellen Gruppe werden die neuen Zwischenprodukte der Formel II einer Cyclisierung unter Ausbildung der 10β,11β-überbrückten Struktur unterworfen.

Die von $R^{2a}$ und K in der Formel II mitumfaßten Hydroxy-, Mercapto- und Keto-Schutzgruppen sind im sauren Milieu leicht abspaltbare Gruppen, wie zum Beispiel die Methoxymethyl-, Ethoxymethyl-, Tetrahydropyranyl-, Ethylendioxyketal-, Ethylendithioketal- oder 2,2-Dimethyltrimethylendioxyketalgruppe.

Schutzgruppen für Amino- und terminale Acetylengruppen, zum Beispiel die Trimethylsilyl- und tert.-Butyldimethylsilylgruppe, sind dem Fachmann ebenfalls bekannt und werden nach der gewünschten Reaktionsfolge auch nach literaturbekannten Verfahren gespalten [Synthesis 1980, 627, J. Org. Chem. 46 (1986) 2280].

Die Cyclisierung der Zwischenprodukte II zu den neuen 10β, 11β-überbrückten Steroiden der allgemeinen Formel IIIa

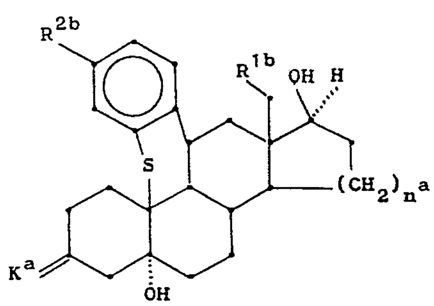

(IIIa),

worin $R^{1b}$, $R^{2b}$, $K^a$ und $n^a$ die gleiche Bedeutung haben wie $R^1$, $R^2$, K, bzw. n, wobei jedoch gegebenenfalls eine in $R^{2b}$ vorhandene Hydroxy-, Mercapto-, Amino-, Oxo- oder terminale Acetylengruppe geschützt ist, die ebenfalls Gegenstand der Erfindung sind, erfolgt durch reduktive radikalische Cyclisierung in einem inerten Lösungsmittel. Als Reduktionsmittel hat sich dabei erfindungsgemäß ein Trialkylzinnhydrid, insbesondere Tributylzinnhydrid, unter Zusatz eines Radikalbildners, beispielsweise Azobisisobutyronitril, bewährt. Als Lösungsmittel ist beispielsweise Toluol geeignet. Die Cyclisierung wird unter Rückfluß und zur Unterstützung der Radikalreaktion unter Bestrahlen mit einer Lichtquelle (z.B. 300W-Glühbirne mit Wolframdraht)

durchgeführt.

Die Umwandlung der so erhaltenen Cyclisierungsprodukte in die letztlich gewünschten Endprodukte der allgemeinen Formel I erfolgt nach an sich bekannten Verfahren, indem man

a) die C-17-Hydroxygruppe gegebenenfalls oxidiert und anschließend

b) gegebenenfalls eine eine Schutzgruppe aufweisende Hydroxygruppe am Phenylenring von dieser Schutzgruppe befreit, gewünschtenfalls aus der Hydroxyverbindung ein entsprechendes Perfluoralkylsulfonat herstellt, gegebenenfalls das Perfluoralkylsulfonat mit einer ent sprechend substituierten Zinntrialkylverbindung in eine Verbindung überführt, die am Phenylenring, gegebenenfalls nach weiteren Reaktionen, das gewünschte Substitutionsmuster aufweist,

oder zuerst b) und dann a) ausführt und danach gegebenfalls

c) den Ring D in gewünschter Weise nach an sich bekannten Methoden funktionalisiert, das so erhaltene Produkt der Einwirkung eines Dehydratationsmittels, das auch zur Freisetzung der 3-Oxogruppe und gegebenenfalls weiterer geschützter Gruppen befähigt ist, unter Ausbildung der 4(5)-Doppelbindung unterwirft

oder

d) das so erhaltene Produkt der Einwirkung eines Dehydratationsmittels, das auch zur Freisetzung der 3-Oxogruppe und gegebenenfalls weiterer geschützter Gruppen befähigt ist, unter Ausbildung der 4(5)-Doppelbindung unterwirft und anschließend, nach Schutz der 3-Oxogruppe, den Ring D in gewünschter Weise funktionalisiert, oder die Schritte a) und b) oder b) und a) nach dem Schritt c) oder d) ausführt,

das so erhaltene Produkt gegebenenfalls von Schutzgruppen befreit, gewünschtenfalls die am Phenylenring gegebenenfalls vorhandene Hydroxy-, Mercapto- und/oder Aminogruppe alkyliert bzw. acyliert, gewünschtenfalls einen Cyanidrest in den Phenylenring einführt, gewünschtenfalls mit Hydroxylamin-hydrochlorid zum Produkt der allgemeinen Formel I mit X in der Bedeutung der Hydroxyiminogruppierung N ~ OH umsetzt sowie gegebenenfalls ein pharmazeutisch verträgliches Säureadditionssalz herstellt.

Im Verlaufe dieser reaktionswege kann es notwendig werden, intermediär erneut Schutzgruppen in Zwischenprodukte, zum Beispiel für in 2 enthaltenen funktionellen Gruppen oder für die 3-Ketogruppe bei anschliessendem Aufbau des Ringes D, einzuführen.

Die für die Herstellung fast aller Endprodukte nötige Oxidation der 17β-Hydroxygruppe wird in an sich bekannter Weise, zum Beispiel durch Oppenauer-Oxidation oder mit Chromsäurereagenzien (Jones' -Reagenz oder Chromsäure-Pyridin) durchgeführt.

Wird das erfindungsgemäße Verfahren nach dem Oxidationsschritt abgebrochen, so werden die 17-Oxidationsprodukte, abgeleitet von der allgemeinen Formel IIIa, erhalten, die ebenfalls Gegenstand der vorliegenden Erfindung sind.

Die Freisetzung der 3-Ketofunktion unter gleichzeitiger Wasserabspaltung und Ausbildung der 4(5)-Doppelbindung erfolgt durch Behandlung mit Säure oder einem sauren Ionenaustauscher. Die saure Behandlung erfolgt in an sich bekannter Weise, indem man das entsprechende 5α-Hydroxy-3-ketal in einem mit Wasser mischbaren Lösungsmittel, wie wäßrigem Methanol, Ethanol oder Aceton, löst und auf die Lösung Katalytische Mengen Mineral- oder Sulfonsäure, wie Salzsäure, Schwefelsäure, Phosphorsäure, Perchlorsäure oder p-Toluolsulfonsäure, oder eine organische Säure, wie Essigsäure, so lange einwirken läßt, bis die Schutzgruppe der 3-Ketofunktion entfernt ist und gegebenenfalls Wasser abgespalten ist. Dabei können auch weitere vorhandene Schutzgruppen teilweise oder vollständig mitabgespalten werden, da das zur Abspaltung eingesetzte saure Agens nicht (unbedingt) selektiv wirkt; gegebenenfalls müssen nach Erzeugung der $\Delta^4$-Doppelbindung und vor Durchführung weiterer reaktionsschritte labile Gruppen wieder neu geschützt werden. Hinzu wird stellvertretend auf den Reaktionsschritt 4D → 4E vor der Oxidation der 17β-OH-Gruppe im Beispiel 4F verwiesen. Die Umsetzung, die bei Temperaturen von 0 bis 100 °C abläuft, kann auch mit einem sauren Ionenaustauscher vorgenommen werden. Der Verlauf der Umsetzung kann mit analytischen Methoden, beispielsweise durch Dünnschichtchromatographie entnommener Proben, verfolgt werden.

Im allgemeinen wird die Schutzgruppenentfernung und Wasserabspaltung in einem Reaktionsschritt bewerkstelligt, indem man das entsprechende 5α-Hydroxy-3-ketal (bzw. 5-en-3-Ketal) in stark saurem Medium eine gewisse Zeit lang reagieren läßt. Genauso gut ist es aber erfindungsgemäß möglich, die Schutzgruppenentfernung und Wasserabspaltung in zwei voneinander getrennten Reaktionsschritten durchzuführen, indem zuerst durch eine kürzere Behandlung des entsprechenden 5α-Hydroxy-3-ketals in mäßig saurem Medium zunächst die entsprechende 5α-Hydroxy-3-keto-Verbindung gewonnen und gegebenenfalls isoliert wird. Die 5α-Hydroxy-3-keto-Verbindung wird dann durch weiteres Einwirkenlassen von Säure unter Wasserabspaltung in die 3-Keto-4-en-Verbindung überführt.

Ein ganz besonderer Vorteil der vorliegenden Erfindung liegt in der großen Bandbreite der am o-Phenylenring einführbaren Substituenten. Zum einen kann der im späteren Endprodukt vorhandene Substituent $R^2$ bereits im Zwischenprodukt der Formel II vorhanden sein, indem er direkt mit dem Thiophenolat über die nucleophile Addition an das Epoxid der Formel IV eingeführt wurde oder nachträglich in der 5-Position des addierten Thiophenolatrestes aufgebaut wurde. Die Anzahl der auf diese Weise herstellbaren, am o-Phenylenring im Endprodukt substituierten Verbindungen ist relativ beschränkt, da nicht alle im Endprodukt wünschenswerten Substituenten die Bedingungen der nucleophilen Addition des Thiophenolats an das jeweilige 5α,10α-Epoxid IV und insbesondere die reduktiven Bedingungen während der Cyclisierung des Zwischenprdukts II zu einem 10β,11β-überbrückten Steroid der allgemeinen Formel III

unbeschadet überstehen.

Zum anderen gelingt es mit einer weiteren Ausführungsform des erfindungsgemäßen Verfahrens, den Substituenten im o-Phenylenring über einen weiten Bereich zu variieren, indem der Substituent erst nach Cyclisierung, und zwar vor oder nach Fertigstellung der Struktur der Ringe A und D eingeführt wird. Dazu wird die im o-Phenylenring vorhandene und geschützte Hydroxygruppe von ihrer Schutzgruppe befreit und aus der freien OH-Verbindung durch Umsetzung mit Perfluoralkylsulfonsäureanhy drid (Alkyl = $C_1$-$C_4$) nach an sich bekannten Methoden [P. J. Stang, M. Hanack and L. R. Subramanian, Synthesis 85 (1982)] die entsprechende Perfluoralkylsulfonatverbindung hergestellt.

Diese Perfluoralkylsulfonatverbindung wird nun durch Reaktion mit einer entsprechend substituierten Zinntrialkylverbindung in eine Verbindung überführt, die dann am Phenylenring entweder bereits das gewünschte Substitutionsmuster besitzt oder dieses Muster wird erst durch weitere Reaktion(en), die an dem über die Zinntrialkylverbindungen eingeführten Substituenten durchgeführt wird (werden), erzeugt. Die dabei erfolgende Verdrängung des Perfluoralkylsulfonatrestes und die Einführung des neuen Substituenten aus der substitutierten Zinntrialkylverbindung wird vorzugsweise übergangsmetallkatalysiert durchgeführt, wobei sich insbesondere Palladium-(0)-Verbindungen bewährt haben, beispielsweise Tetrakis(triphenylphosphin)-palladium(0) (J.E. McMurry and S. Mohanraj, Tetrahedron Letters, 24, No. 27, S. 2723-2726, 1983; X. Lu und J. Zhu, Communications, S. 726-727, 1987; Q.-Y. Chen und Z.-Y. Yang, Tetrahedron Letters 27, No. 10, S. 1171-1174, 1986; S. Cacchi, P.G. Ciattini, E. Morera und G. Ortar, Tetrahedron Letters 27 No. 33, S. 3931-3934, 1986; A.M. Echavarren und J.K. Stille, J. Am. Chem. Soc. 1987, 109, S. 5478-5486)

Im Beispiel 4 ist stellvertretend eine Reaktionsfolge aufgezeigt, bei welcher zunächst aus der Trifluormethan-sulfonat-Verbindung durch Umsetzung mit (1-Ethoxyvinyl)-tributylzinn unter Zusatz von Tetrakis-(triphenylphosphin)-palladium(0) die entsprechende (1-Ethoxyvinyl)-Verbindung hergestellt und diese anschließend durch saure Spaltung und Umlagerung in die entsprechende Acetylverbindung überführt wird. Nach erneutem Schutz der dabei freigesetzten 3-Ketofunktion und der neugebildeten Ketofunktion im Acetylsubstituenten in 5-Stellung des o-Phenylenthio-Restes kann die 17β-OH-Gruppe oxidiert und an die dabei entstandene Ketogruppe nucleophil addiert werden.

Mit dem erfindungsgemäßen Verfahren ist es aber auch möglich, den am Phenylenring gewünschten Substituenten direkt einzuführen, beispielsweise den Vinylsubstitutenten durch Umsetzung der Trifluormethansulfonat-Verbindung mit (Vinyl)-tributylzinn.

Weitere Beispiele für Reaktionen, die an der Trifluormethansulfonat-Gruppe möglich sind, finden sich in der Europäischen Patentanmeldung EP-A Veröffentlichungsnummer 0283428 (entspricht PCT/DE 88/00150) und vor allem in den oben genannten Veröffentlichungen.

Edukte mit einem D-Homo-Steroidgerüst sind z.B. durch Tiffeneau-Umlagerung analog der in Australian J. Chem. 8 (1955), 519 und in "Organic Reactions in Steroid Chemistry" Vol. 2, 388 veröffentlichten Vorschrift zu erhalten. Die notwendigen 17α-Aminomethyl-17β -hydroxyverbindungen sind zum Beispiel über die Öffnung der 17,20-Spiroepoxide mit Ammoniak oder auch durch Lithiumaluminiumreduktion der acetylierten 17β-Hydroxy-17α-cyanoverbindungen zugänglich. Die Spiroepoxide sind durch Umsatz der entsprechenden 17-Ketone mit Dimethylsulfoniummethylid in Dimethylformamid [Journal f. prakt. Chemie 314 (1972), 667-668] zugänglich. Die acetylierten Cyanhydrine sind durch Anlagerung von Cyanwasserstoff an die entsprechenden 17-Ketone und anschließende Acetylierung nach bekannten Vorschriften [z.B. Australian J. Chem. 8 (1955), 519] zugänglich.

Edukte mit einem ungesättigten D-Ring sind zum Beispiel durch modifizierte Saegusa-Oxidation [Tetrahedron 42 (1986) 2971] der entsprechenden Enolverbindungen des 17-Ketons zugänglich. Zum Beispiel ist der Trimethylsilylenolether durch Überführung des 17-Ketons mit Lithiumdiisopropylamid in Tetrahydrofuran in das korrespondierende Enolat und Abfang durch Trimethylchlorsilan darstellbar (Synthesis 1983, 1).

Die Einführung der Substituenten $R^5$ und $R^6$ erfolgt nach den üblichen Verfahren des C-17-Seitenkettenaufbaus durch nucleophile Addition an das durch z.B. Oppenauer-Oxidation der C-17-Hydroxygruppe erhaltene 17-Keton und Folgereaktionen ("Terpenoids and Steroids", Specialist Periodical Report, The Chemical Society, London, Vol. 1-12).

Die Einführung des Substituenten -C≡C-U als $R^6$, wobei U die oben angegebene Bedeutung hat, erfolgt mit Hilfe einer Verbindung der allgemeinen Formel MC≡C-U′ in der U′ der mit einer Schutzgruppe, wie zum Beispiel Trimethylsilyl oder tert.-Butyldimethylsilyl, geschützte Rest U oder aber im Fall, wenn U eine Alkylgruppe mit 1 - 4 C-Atomen ist, U′ selbst der Rest U ist.

Die metallorganische Verbindung kann auch in situ gebildet und mit dem 17-Keton zur Reaktion gebracht werden. So kann man zum Beispiel auf das 17-Keton in einem geeigneten Lösungsmittel Acetylen und ein Alkalimetall, insbesondere Kalium, Natrium oder Lithium, in Gegenwart eines Alkohols oder in Gegenwart von Ammoniak einwirken lassen. Das Alkalimetall kann auch in Form von zum Beispiel Methyl- oder Butyllithium zur Einwirkung kommen. Als Lösungsmittel sind insbesondere Dialkylether, Tetrahydrofuran, Dioxan, Benzol und Toluol geeignet.

Die Einführung von 3-Hydroxypropin, -propen bzw. -propan in 17-Stellung erfolgt durch Umsetzung des 17-Ketons mit dem Dianion des Propargylalkohols (3-Hydroxypropin), zum Beispiel dem in situ generierten Dikaliumsalz des Propargylalkohols, zum 17α-(3-Hydroxyprop-1-inyl)-17β-hydroxyderivat oder mit metallierten Derivaten des 3-Hydroxypropins, zum Beispiel mit 1-Lithium-3-(tetrahydropyran-2′-yloxy)-prop-1-in-1-id, zum 17α-[3-(Tetrahydropyran-2′-yloxy)-prop-1-inyl]-17β-hydroxyderivat, die anschließend zu den 17-(3-Hydroxypropyl- bzw. Hydroxypropenyl)17β-hydroxy-Verbindungen hydriert werden können. Das gelingt zum Beispiel

durch Hydrierung bei Raumtemperatur und Normaldruck in Lösungsmitteln wie Methanol, Ethanol, Propanol, Tetrahydrofuran (THF) oder Essigester unter Zusatz von Edelmetall-Katalysatoren wie Platin oder Palladium.

Die Einführung homologer Hydroxyalkin-, Hydroxyalken- und Hydroxyalkangruppen erfolgt in entsprechender Weise mit Homologen des Propargylalkohols

Die Verbindung mit der Z-konfigurierten Doppelbindung in der Hydroxypropenylgruppe entsteht durch Hydrieren der acetylenischen Dreifachbindung mit einem desaktivierten Edelmetallkatalysator (J. Fried, J.A. Edwards: Organic Reactions in Steroid Chemistry, Van Nostrand Reinhold Company 1972, Seite 134; und H.O. House: Modern Synthetic Reactions 1972, Seite 19) Als desaktivierte Edelmetallkatalysatoren kommen beispielsweise 10 % Palladium auf Bariumsulfat in Gegenwart eines Amins oder 5 % Palladium auf Calciumcarbonat unter Zusatz von Blei(II)-acetat infrage. Die Hydrierung wird nach der Aufnahme von einem Äquivalent Wasserstoff abgebrochen.

Die Verbindung mit der E-konfigurierten Doppelbindung in der Hydroxypropenylgruppe entsteht durch Reduktion der acetylenischen Dreifachbindung in an sich bekannter Weise. In der Literatur sind eine ganze Reihe von Methoden zur Umwandlung von Alkinen in trans-Olefine beschrieben, beispielsweise die Reduktion mit Natrium in flüssigem Ammoniak [J. Am. Chem. Soc. 63 (1941) 216], mit Natriumamid in flüssigem Ammoniak [J. Chem. Soc. (1955), 3558], mit Lithium in niedermolekularen Aminen [J. A. Chem. Soc. 77 (1955) 3378], mit Boranen [J. Am. Chem. Soc. 93 (1971) 3395 und 94 (1972) 6560], mit Diisobutylaluminiumhydrid und Methyl-Lithium [J. Am. Chem. Soc. 89 (1967) 4245). Eine weitere Möglichkeit ist die Reduktion der Dreifachbindung mit Chrom(II)-sulfat in Gegenwart von Wasser oder Dimethylformamid in schwach saurem Milieu [J. Am. Chem. Soc. 86 (1964) 4358] sowie allgemein die Reduktion durch Einwirkung von Übergangsmetallverbindungen unter Wechsel der Oxydationsstufe.

Die Einführung der Hydroxyalkene kann auch direkt erfolgen durch Addition einer entsprechenden metallierten Hydroxyalkenylverbindung, wie zum Beispiel 1-Lithium-3-(tetrahydropyran-2′-yloxy)-prop-1(E)-en (J. Org. Chem. 40, 2265) oder 1-Lithium-3-(tetrahydropyran-2′-yloxy)-prop-1 (Z)-en (Synthesis 1981, 999). Homologe können auf diese Art ebenfalls eingeführt werden.

Die Einführung von 3-Hydroxypropan in 17-Stellung kann ebenfalls direkt durch Umsetzung des 17-Ketons mit metallierten Derivaten von 3-Halogenpropanolen - wobei die Hydroxygruppe im Metallierungsschritt als Alkoholat (Tetrahedron Letters 1978, 3013) oder als geschützte Funktion (J. Org. Chem. 37 1947) vorliegt - zu der 17-(3-Hydroxypropyl)-17β-hydroxy-Verbindung bzw. zu der an der terminalen Hydroxygruppe geschützten Verbindung erfolgen. Als Schutzgruppen kommen zum Beispiel die Ethoxyethyl-, Tetrahydropyranyl- und Methoxymethyl-Gruppen infrage.

Werden Endprodukte der Formel I gewünscht mit $R^5/R^6$ in der Bedeutung von

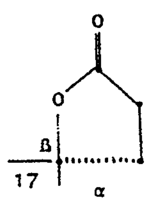

so wird die 17-(3-Hydroxypropyl)-Verbindung in an sich bekannter Weise oxydiert, zum Beispiel mit Jones'-Reagenz, Braunstein, Pyridiniumdichromat, Pyridiniumchlorochromat, Chromsäure-Pyridin oder dem Fetizon-Reagenz Silbercarbonat/Celite [Compt. rend. 267 (1968) 900].

Die Darstellung von Endprodukten der Formel I mit $R^5/R^6$ in der Bedeutung von

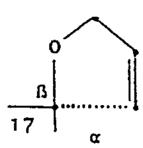

erfolgt durch Ringschlußreaktion des entsprechenden 17-(3-Hydroxyprop1-(Z)-enyl-17β-hydroxy-Eduktes.

Der Aufbau der 17-Cyanmethylseitenkette erfolgt in an sich bekannter Weise aus dem 17-Keton zum Beispiel über das 17-Spiroepoxid und Spaltung des Spiroepoxids mit HCN gemäß Z. Chem, 18 (1978) 259-260.

Auch die Einführung der 17-Hydroxyacetylseitenkette erfolgt nach an sich bekannten Methoden, beispielsweise nach den in J. Org. Chem. 47 (1982), 2993-2995, Chem. Ber. 113 (1984), 1184 bzw. US-Patent 4,600,538 beschriebenen Methoden.

Zur Einführung der Gruppierungen

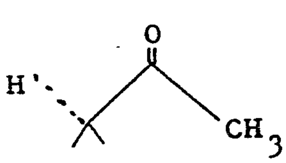

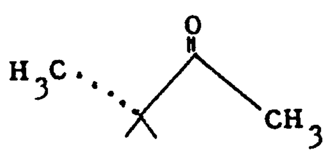

wird das 17-Keton mit Tosylmethylisocyanid (Chem. Ind. 1972, 213) in die 17-Nitrilverbindung (Tetrahedron 31 (1975), 2151) überführt, das direkt mit Methyllithium oder Methylmagnesiumbromid in die 17-Acetylverbindung umgewandelt werden kann, welche nach Enolisierung mit K-tert.-Butylat in Tetrahydrofuran und Umsetzung mit Methyljodid die gewünschte 17α-Methyl-17β-acylgruppierung liefert. Diese Sequenz Methyladdition an das Nitril und anschließende Alkylierung kann auch in umgekehrter Folge ausgeführt werden.

In Z bzw. am Phenylenring vorhandene freie Hydroxy- bzw. Mercapto-und/oder Aminogruppen können in an sich bekannter Weise alkyliert oder acyliert werden.

Neben der bereits beschriebenen Methode können Verbindungen mit einem Dialkylamin-Substituenten am Phenylenring durch Reaktion mit Bromcyan in aprotischen Lösungsmitteln, wie zum Beispiel Dioxan, Benzol oder Toluol, bei erhöhter Temperatur (Amin-Abbau nach Braun) analog den zum Beispiel in Org. Reactions 7, 198 (1953), K.W. Bentley, Techniques of Organic Chemistry 11, 773 (1963) und Houben Weyl, 5/4, 151 (1960) angegebenen Vorschriften in guter Ausbeute in die entsprechenden (N-Cyan-N-alkylaminoaryl)-Derivate überführt werden.

Die erhaltenen Verbindungen der allgemeinen Formel I mit X in der Bedeutung eines Sauerstoffatoms können gewünschtenfalls durch Umsetzung mit Hydroxylaminhydrochlorid in Gegenwart von tertiären Aminen bei Temperaturen zwischen -20 und +40 °C in die Oxime (Formel I mit X in der Bedeutung der Hydroxyiminogruppierung N~OH, wobei die Hydroxygruppe syn-oder antiständig sein kann) überführt werden. Geeignete tertiäre Basen sind beispielsweise Trimethylamin, Triäthylamin, Pyridin, N,N-Dimethylaminopyridin, 1,5-Diazabicyclo[4.3.0]nonen-5 (DBN) und 1,5-Diazabicyclo[5.4.0]undecen-5 (DBU), wobei Pyridin bevorzugt ist.

Die neuen Verbindungen der allgemeinen Formel I sowie deren pharmazeutisch verträgliche Additionssalze mit Säuren sind wertvolle Pharmaka. So verfügen sie über eine starke Affinität zum Gestagenrezeptor und besitzen einen überraschend großen Bereich an antigestagenen und antiglucocorticoiden Eigenschaften. Diese wichtigen biologischen Wirksamkeiten können für medizinische Zwecke genutzt werden.

Wirkstoffe dieser Art mit ausgeprägter antigestagener Aktivität sind zur Auslösung von Aborten geeignet, da sie das zur Aufrechterhaltung der Schwangerschaft erforderliche Progesteron vom Rezeptor verdrängen. Sie sind deshalb wertvoll und interessant im Hinblick auf ihre Verwendung zur postcoitalen Fertilitätskontrolle.

Sie können auch gegen hormonelle Unregelmäßigkeiten, zur Menstruationsauslösung und zur Geburtseinleitung eingesetzt werden.

Außerdem können sie für die Behandlung von hormonabhängigen Carcinomen eingesetzt werden.

Die erfindungsgemäßen Verbindungen der allgemeinen Formel I sowie deren pharmazeutisch verträgliche Additionssalze mit Säuren weisen auch eine antiglucocorticoide Aktivität auf und können somit auch als Arzneimittel zur Therapie corticoid-induzierter Störungen (Glaukom) sowie zur Bekämpfung von Nebenwirkungen, die bei langfristiger Behandlung mit Glucocorticoiden auftreten (Cushing-Syndrom) eingesetzt werden. Sie ermöglichen daher auch die auf eine Supersekretion der Glucocorticoide zurückzuführenden Störungen, vor allem die Adipositas, Arteriosklerose, Hypertension, Osteoporose, Diabetes sowie die Insomnie zu bekämpfen.

Die Erfindung betrifft somit auch Arzneimittel auf Basis der pharmazeutisch verträglichen, d.h. in den verwendeten Dosen nicht toxischen Verbindungen der allgemeinem Formel I sowie deren pharmazeutisch verträgliche Additionssalze mit Säuren, gegebenenfalls zusammen mit den üblichen Hilfs- und Trägerstoffen.

Die erfindungsgemäßen Verbindungen und deren Salze können nach an sich bekannten Methoden der Galenik zu pharmazeutischen Präparaten für die enterale, perkutane, Parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen, Salben, Cremes und Gelen verabreicht werden.

Der oder die Wirkstoffe können dabei mit den in der Galenik üblichen Hilfsstoffen wie z.B. Gummiarabikum, Talk, Stärke, Mannit, Methylcellulose, Laktose, Tensiden wie Tweens(R) oder Myrj(R) Magensiumstearat, wäßrigen oder nichtwäßrigen Trägern, Paraffinderivaten, Netz-, Dispergier-, Emulgier-, Konservierungsmitteln und Aromastoffen zur Geschmackskorrektur (z.B. ätherischen Ölen) gemischt werden.

Die Erfindung betrifft somit auch pharmazeutische Zusammensetzungen, die als Wirkstoff zumindest eine erfindungsgemäße Verbindung oder eines ihrer pharmazeutisch verträglichen Additionssalze mit Säuren enthalten.

Als Additionssalze der erfindungsgemäßen Produkte mit Säuren sind insbesondere die Hydrochloride und die Methansulfonate zu nennen.

Eine Dosiseinheit enthält etwa 1 - 100 mg Wirkstoff(e).

Die Dosierung der erfindungsgemäßen Verbindungen liegt beim Menschen bei etwa 1 - 1000 mg pro Tag.

Stellvertretend für alle erfindungsgemäßen Verbindungen wurde zur Kennzeichnung der antigestagenen Wirkung die abortive Wirkung der Verbindung 6 c (siehe exp. Teil) bestimmt:

Die Versuche wurden an weiblichen Ratten im Gewicht von ca. 200 g durchgeführt. Nach erfolgter Anpaarung wurde der Schwangerschaftsbeginn durch Nachweis von Spermien in Vaginalabstrichen gesichert. Der Tag des Spermiennachweises gilt als Tag 1 der Grvidität (= d1 p.C.).

Die Behandlung der Tiere mit der zu testenden Substanz bzw. dem Lösungsmittel erfolgte nach der Nidation der Blastocysten von d5 p.c. bis d7 p.c. An d9 p.c. wurden die Tiere getötet und die Uteri auf Implantate und Resorptionsstellen hin untersucht. Von allen Uteri wurden Fotos angefertigt. Das Fehlen von Implantaten, pathologische, hämorrhagische oder sonst abnorme Nidationsstellen, wurde als Abort gewertet.

Die Testsubstanz wurde in einem Benzylbenzoat-Rizinusöl-Gemisch (Verhältnis 1 + 4) gelöst. Das Vehikelvolumen pro Einzeldosis betrug 0,2 ml. Die Behandlung erfolgte subcutan.

Bei einer Dosis von 3,0 mg/Tier/Tag war diese Verbindung abortiv voll wirksam (n Abort/n behandelten Versuchstieren 4/4; Lösungsmittelkontrolle 0/4).

Die Säulenchromatographischen Reinigungsschritte in den nachfolgenden Beispielen werden, soweit nicht anders erwähnt, an Aluminiumoxid der Aktivitätsstufe III mit Hexan/Essigester, gegebenenfalls mit einem Gemisch steigender Polarität, durchgeführt.

**Beispiel 1**

Darstellung von 17β-Hydroxy-10β,11β(5-methoxy-o-phenylenthio)-estr-4-en-3-on (1C)

**A.** 10β-(2-Brom-5-methoxy-phenylthio)-3,3-(2,2-dimethyltrimethylendioxy)-estr-9(11)-en-5α,17β-diol (1A)

22,3g 2-Brom-5-methoxythiophenol werden in 182 ml absolutem THF gelöst und bei -40 °C mit 40 ml einer 2,5 molaren n-Butyllithium-Lösung in Hexan versetzt. Es wird die Kühlung entfernt und 30 Minuten gerührt. Nun werden 12,73 g 3,3-(2,2-Dimethyltrimethylendioxy)-5α,10α-epoxy-estr-9(11)-en-17β-ol gelöst in 100 ml absolutem THF zugetropft, wobei die Temperatur bei 0 °C gehalten wird. Anschließend wird 2 Stunden bei Raumtemperatur nachgerührt.

Die Reaktionsmischung wird mit 300 ml gesättigter $NH_4Cl$-Lösung versetzt und mit Essigester extrahiert, die organische Phase mit Wasser gewaschen und über $Na_2SO_4$ getrocknet, das Lösungsmittel im Vakuum abgezogen.

Säulenchromatographie ergibt 17,99 g 1A;

Schmelzpunkt: 117 - 119 °C

**B.** 3,3-(2,2-Dimethyltrimethylendioxy)-10β,11β-(5-methoxy-o-phenylenthio)-estran-5α,17β-diol (1B)

2,3 g 1A, 1 ,25 g Tributylzinnhydrid und 25 mg Azobisisobutyronitril werden in 50 ml Toluol p.A. unter Argon und Rückfluß 1 Stunde lang im Licht einer 300W-Glühbirne gerührt.

Säulenchromatographie ergibt 1,09 g 1B;

Schmelzpunkt: 95 - 97 °C

**C.** 17β-Hydroxy-10β,11β-(5-methoxy-o-phenylenthio)-estr-4-en-3-on (1C)

600 mg (1,2mMol) 1B und 1,1 ml 4nHCl werden in 22 ml Aceton p.A. 1 ,5 Stunden unter Rückfluß gerührt.

Die Reaktionslösung wird mit $NaHCO_3$-Lösung und Essigester versetzt, die organische Phase abgetrennt, die wäßrige Phase mit Essigester gewaschen, die vereinigten organischen Phasen mit Wasser gewaschen und über Natriumsulfat getrocknet, das Lösungsmittel im Vakuum abgezogen.

Säulenchromatographie ergibt 364 mg 1C;

Schmelzpunkt: 85 - 88 °C

**Beispiel 2**

Darstellung von 17β-Hydroxy-10β,11β-(5-methoxy-o-phenylenthio)-17α-(1-proninyl)-estr-4-en-3-on (2C)

**A.** 3,3-(2,2-Dimethyltrimethylendioxy)-5α-hydroxy-10β,11β-(5-methoxy-o-phenylenthio)-estran-17-on (2A)

1,24 g 1B werden in 52 ml Toluol p.A. gelöst, mit 174 mg Aluminiumtriisopropylat und 1,1 ml Cyclohexanon versetzt und 4,5 Stunden unter Rückfluß am Wasserabscheider erhitzt. Gegebenenfalls wird weiteres Äquivalent Aluminiumtriisopropylat hinzugefügt und einige Stunden weitergerührt.

Die Reaktionslösung wird mit Wasser verdünnt, mit Essigester extrahiert, die organische Phase mit Wasser gewaschen, über Natriumsulfat getrocknet und im Vakuum eingeengt.

Säulenchromatographie ergibt 0,82 g 2A;

Schmelzpunkt: 249 - 250 °C

**B.**

3,3-(2,2-Dimethyltrimethylendioxy)-10β,11β-(5-methoxy-o-phenylenthio)-17α-(1-Propinyl)-estran-5α,17β-diol (2B)

Durch 11 ml absolutes THF wird bei 0 °C während 1 ,5 Stunden ein Strom von Propin geleitet; zu dieser Lösung tropft man 3 ml n-Butyllithium (2,5M in Hexan), rührt 90 Minuten bei 0 °C, fügt 640 mg (1 ,25mMol) 2A in 30 ml absoluten THF hinzu und rührt eine weitere Stunde bei Raumtemperatur.

Die Reaktionslösung wird mit 25 ml Wasser versetzt, mit Essigester extrahiert, die organische Phase mit Wasser gewaschen, über Natriumsulfat getrocknet und im Vakuum eingeengt.

Ausbeute: 690 mg 2B als Schaum.

**C.** 17β-Hydroxy-10β,11β-(5-methoxy-o-phenylenthio)-17α-(1-proninyl)-estr-4-en-3-on (2C)

690 mg 2B werden in 25 ml Aceton p.A. gelöst und mit 1,27 ml 4nHCl 4,5 Stunden bei 60 °C gerührt.

Die Reaktionslösung wird mit gesättigter $NaHCO_3$-Lösung versetzt, mit Essigester extrahiert, die organische Phase mit Wasser gewaschen, über Natriumsulfat getrocknet, das Lösungsmittel im Vakuum

entfernt.
Säulenchromatographie ergibt 330 mg 2C;
Schmelzpunkt: 254 - 257 °C

**Beispiel 3**

Darstellung von 10β,11β-(5-Diethylamino-o-phenylenthio)-17β-hydroxy-17α-(1-proninyl)-estr-4-en-3-on (3K)

**A.** 10β-(3-Aminophenylthio)-3,3-(2,2-dimethyltrimethylendioxy)-estr-9(11)-en-5α,17β-diol (3A)
Die Durchführung und Aufarbeitung erfolgt wie bei Beispiel 1A beschrieben.
Eingesetzte Mengen: 500 mg 3,3-(2,2-Dimethyltrimethylendioxy)-5α,10α-epoxy-estr-9(11)-en-17β-ol (in 4 ml absolutem THF), 750 mg 3-Mercaptoanilin (in 5 ml absolutem THF), 3,73 ml 1,6M n-Butyllithium in Hexan.
Ausbeute nach Säulenchromatographie: 484 mg 3A als Schaum.

**B.** 17β-Acetoxy-10β-(3-acetylaminophenylthio)-3,3-(2,2-dimethyltrimethyl endioxy)-estr-9(11)-en-5α-ol (3B)
7,3g 3A und 44 ml Acetanhydrid werden in 73 ml Pyridin p.A. bei Raumtemperatur 4 Stunden lang unter Argon gerührt.
Die Reaktionslösung wird mit Methylenchlorid verdünnt, mit $NaHCO_3$-Lösung versetzt, die organische Phase abgetrennt, mit Wasser gewaschen und über Natriumsulfat getrocknet, das Lösungsmittel wird im Vakuum abgezogen.
Ausbeute nach Säulenchromatographie: 7,65 g 3B als Schaum.

**C.** 3,3-(2,2-Dimethyltrimethylendioxy)-10β-(3-ethylaminophenylthio)-estr-9(11)-en-5α,17β-diol (3C)
7 g 3B und 2,9 g Lithiumaluminiumhydrid werden in 250 ml absolutem Ether 17 Stunden bei 50 °C unter Argon am Rückfluß gerührt.
Das überschüssige Lithiumaluminiumhydrid wird unter Eiskühlung mit Wasser hydrolysiert, die organische Phase abgetrennt, die wäßrige Phase mit Essigester gewaschen, die vereinigten organischen Phasen mit Wasser gewaschen und über Natriumsulfat getrocknet, das Lösungsmittel abgezogen.
Ausbeute nach Säulenchromatographie: 5,37 g 3C;
Schmelzpunkt: 100 °C

**D.**
17β-Acetoxy-10β-(3-N-acetyl-N-ethylaminophenylthio)-3,3-(2,2-dimethyltrimethylendioxy)-estr-9(11)-en-5α-ol (3D)
Durchführung und Aufarbeitung wie bei Beispiel 3B.
Eingesetzte Mengen: 5,3 g 3C 38 ml Acetanhydrid, 53 ml Pyridin p.A.
Ausbeute nach Säulenchromatographie: 4,76 g 3D;
Schmelzpunkt: 188 °C

**E.** 10β-(3-Diethylaminophenylthio)-3,3-(2,2-dimethlyltrimethylendioxy)-estr-9(11)-en-5α,17β-diol (3E)
Durchführung und Aufarbeitung wie bei Beispiel 3C.
Eingesetzte Mengen: 3,2 g 3D, 1,4 g Lithiumaluminiumhydrid, 100 ml THF, 20 ml Ether.
Ausbeute nach Säulenchromatographie: 2,72 g 3E;
Schmelzpunkt: 104 °C

**F.** 10β-(2-Brom-5-diethylaminophenylthio)-3,3-(2,2-dimethyltrimethylendioxy)-estr-9(11)-en-5α,17β-diol (3F)
556 mg 3E und 175 mg N-Bromsuccinimid werden in 6 ml $CCl_4$ 2 Stunden bei 0 °C unter Argon gerührt.
Anschließend wird mit $NaHCO_3$-Lösung versetzt, in Methylenchlorid aufgenommen, die organische Phase abgetrennt, die wäßrige Phase mit Methylenchlorid gewaschen, die vereinigten organischen Phasen mit Wasser gewaschen und über Natriumsulfat getrocknet; das Lösungsmittel wird abgezogen.
Ausbeute nach Säulenchromatographie: 510 mg 3F
; Schmelzpunkt: 115 °C

**G.** 10β,11β-(5-Diethylamino-o-phenylenthio)-3,3-(2,2-dimethyltrimethylendioxy)-estran-5α,17β-diol (3G)
Durchführung und Aufarbeitung wie bei Beispiel 1B.
Eingesetzte Mengen: 250 mg 3F, 126 mg Tributylzinnhydrid und 2 mg Azobisisobutyronitril in 16 ml Toluol p.A.
Ausbeute: 102 mg 3G als weißer Schaum

**H.** 10β,11β-(5-Diethylamino-o-phenylenthio)-3,3-(2,2-dimethyltrimethylendioxy)-5α-hydroxy-estran-17-on (3H)
Durchführung und Aufarbeitung wie bei Beispiel 2A.
Eingesetzte Mengen: 555 mg 3G in 25 ml Toluol p.A., 100 mg Aluminiumtriisopropylat, 0,46 ml Cyclohexanon.
Ausbeute nach Säulenchromatographie: 430 mg 3H:
Schmelzpunkt: 271 °C

I. 10β,11β-(5-Diethylamino-o-phenylenthio)-3,3-(2,2-dimethyltrimethylendioxy)-17α-(1-propinyl)-estran-5α,17β-diol (3I)

Durchführung und Aufarbeitung wie bei Beispiel 2B.

Eingesetzte Mengen: 860 mg 3H in 20 ml absoluten THF, 50 ml absoluten THF, 2 Stunden Propin-Strom, 3,7 ml n-Butyllithium (2,5M in Hexan).

Ausbeute: 880 mg 3I als Schaum.

K. 10β,11β-(5-Diethylamino-o-phenylenthio)-17β-hydroxy-17α-(1-propinyl)-estr-4-en-3-on (3K)

Durchführung und Aufarbeitung wie bei Beispiel 2C.

Eingesetzte Mengen: 880 mg 3I, 28 ml Aceton p.A., 1,4 ml 4N HCl.

Ausbeute: 548 mg 3K als Schaum

$^1$H-NMR-Spektrum($CD_2Cl_2$, 300 MHz): δ (in ppm) 0,45 (s,H-18), 1,90 (s,H-22), 3,30 (q, N-$CH_2$-$CH_3$), 1,15 (t, N-$CH_2$-$CH_3$), 3,40 (ddbr, H-11), 5,90 (sbr, H-4), 7,25 (d, H-3′), 6,45 (dd, H-4′), 6,3 (d, H-6′).

**Beispiel 4**

Darstellung von 10β,11β-(5-Acetyl-o-phenylenthio)-17β-hydroxy-17α-(1-propinyl)-estr-4-en-3-on (4H)

A. 3,3-(2,2-Dimethyltrimethylendioxy)-10β,11β-(5-hydroxy-o-phenylenthio)-estran-5α,17β-diol (4A)

500 mg 1B werden in 5 ml destilliertem Dimethylformamid gelöst und mit 293 mg Natriummethanthiolat 4 Stunden bei 160 °C unter Rückfluß gerührt. Die Reaktionslösung wird mit gesättigter $NaHCO_3$-Lösung versetzt und mit Methylenchlorid extrahiert, die organische Phase mit Wasser gewaschen und über Natriumsulfat getrocknet, das Lösungsmittel im Vakuum entfernt. Ausbeute nach Säulenchromatographie an $SiO_2$ mit Hexan/Essigester:

330 mg 4A

Schmelzpunkt: 271 °C

B. 3,3-(2,2-Dimethyltrimethylendioxy)-10β,11β-(5-trifluormethylsulfonyloxy-o-phenylenthio)-estran-5α,17β-diol (4B)

2,53 g 4A und 3,43 g Dimethylaminopyridin werden in 75 ml Methylenchlorid gelöst und bei -30 °C unter Argon mit 0,12 ml Trifluormethansulfonsäureanhydrid versetzt. Es wird 10 Minuten nachgerührt. Die Reaktionslösung wird in Wasser gegossen und mit Methylenchlorid extrahiert, die organische Phase mit Wasser gewaschen und über Natriumsulfat getrocknet, das Lösungsmittel im Vakuum abgezogen.

Ausbeute nach Säulenchromatographie an $SiO_2$ mit Hexan/Essigester:

2,56 g 4B

Schmelzpunkt: 189 °C

C. 3,3-(2,2-Dimethyltrimethylendioxy)-10β,11β-[5-(1-ethoxyethenyl)-o-phenylenthio]-estran-5α,17β-diol (4C1) und 10β,11β-(5-Acetyl-o-phenylenthio)-3,3-(2,2-dimethyltrimethylendioxyestran-5α,17β-diol (4C2)

2,55 g 4B werden in 60 ml Dimethylformamid gelöst, mit 338 mg LiCl, 1,51 ml (1-Ethoxyvinyl)-tributylzinn und 95 mg Tetrakis-(triphenylphosphin)-palladium(0) versetzt und bei 110 °C 2h unter Argon gerührt.

Nach dem Erkalten wird die Reaktionslösung auf gesättigte $NaHCO_3$-Lösung gegossen, mit Essigester extrahiert, mit gesättigter NaCl-Lösung gewaschen und über $Na_2SO_4$ getrocknet.

Ausbeute: 2,5 g eines Gemisches aus 4C1 und 4C2 im Verhältnis von ca. 1:2 als Schaum.

4C2 entsteht dabei während der Aufarbeitung und dem säulenchromatographischen Reinigungsschritt aus 4C1.

D. 10β,11β-(5-Acetyl-o-phenylenthio)-17β-hydroxy-estr-4-en-3-on (4D)

2,24 g des Gemisches aus 4C1 und 4C2 (Verh. ca. 1:2) werden in 92 ml Aceton p.a. gelöst, mit 1,6 ml konz. HCl versetzt und 30 min bei 60 °C gerührt. Anschließend wird die Reaktionslösung mit gesättigter $NaHCO_3$-Lösung versetzt, in Essigester aufgenommen, über $Na_2SO_4$ getrocknet und am Rotationsverdampfer eingeengt..

Ausbeute nach Säulenchromatographie an $SiO_2$ mit Hexan/Essigester: 1,02 g 4D (Schaum)

E. 3,3-(2,2-Dimethyltrimethylendioxy)-10β,11β-[5-[1,1-(2,2-dimethyltrimethylendioxy)-ethyl]-o-phenylenthiol]-ester-5-en-17β-ol (4E)

1,15 g 4D werden in 30 ml Toluol p.a. gelöst, mit 2,82 g 1,3-Dimethylpropandiol und 206 mg p-Toluolsulfonsäure versetzt und 2h unter Argon am Wasserabscheider unter Rückfluß erhitzt.

Nach dem Erkalten wird die Reaktionslösung mit 1N NaOH versetzt, in Essigester aufgenommen, mit gesättigter $NaHCO_3$ gewaschen, über $Na_2SO_4$ getrocknet und am Rotationsverdampfer eingeengt.

Ausbeute nach Säulenchromatographie: 1,5 g

Schmelzpunkt: 177 °C

**F.**
3,3-(2,2-Dimethyltrimethylendioxy)-10β,11β-[5-[1,1-(2,2-dimethyltrimethylendioxy)-ethyl]-o-phenylenthiol]-estr-5-en-17-on (4F)

Durchführung und Aufarbeitung wie bei Beispiel 2A.

Eingesetzte Mengen: 1,5 g 4E, 70 ml Toluol p.a., 1,95 ml Cyclohexanon, 300 mg Aluminiumtriisopropylat.
Ausbeute nach Säulenchromatographie: 1,2 g 4F
Schmelzpunkt: 169 °C

**G.**
3,3-(2,2-Dimethyltrimethylendioxy)-10β,11β-[5-[1,1-(2,2-dimethyltrimethylendioxy)-ethyl]-o-phenylenthiol]-17α-(1-propinyl)-estr-5-en-17β-ol (4G)

Durchführung und Aufarbeitung wie bei Beispiel 2B.

Eingesetzte Mengen: 17 ml THF abs., 2h Propin-Strom, 3,8 ml n-Butyllithium (1,6M in Hexan), 600 mg 4F in 17 ml THF abs..
Ausbeute: 620 mg 4G
Schmelzpunkt: 186 °C

**H.** 10β,11β-(5-Acetyl-o-phenylenthio)-17β-hydroxy-17α-(1-propinyl)-estr-4-en-3-on (4H)

Durchführung und Aufarbeitung wie bei Beispiel 2C.

Eingesetzte Mengen: 620 mg 4G, 23 ml Aceton p.a., 1,55 ml 4N HCl.
Ausbeute: 347 mg 4H (nach Säulenchromatographie)
Schmelzpunkt: 174 °C

**Beispiel 5**

Darstellung von 10β,11β-(5-Dimethylamino-o-phenylenthio)-17β-hydroxy-17α-(1-propinyl)-estr-4-en-3-on (5I)

**A.** 3,3-(2,2-Dimethyltrimethylendioxy)-10β-(3-formylamino-phenylthio)-estr-9(11)-en-5α,17β-diol (5A)

0,4 ml Acetanhydrid und 0,17 ml HCOOH werden zusammengegeben und bei Raumtemperatur unter Argon 15 min gerührt. Zu dieser Lösung fügt man 1,0 g 3A gelöst in 5 ml Pyridin p.a. hinzu und rührt bei Raumtemperatur unter Argon 2h nach.

Anschließend wird das Reaktionsgemisch in Essigester aufgenommen, mit gesättigter $NaHCO_3$-Lösung geschüttelt bis ein pH-Wert von ca. 8 erreicht ist, mit Wasser gewaschen, über $Na_2SO_4$ getrocknet und am Rotationsverdampfer eingeengt.
Ausbeute nach Säulenchromatographie: 935 mg 5A, Schaum

**B.** 3,3-(2,2-Dimethyltrimethylendioxy)-10β-(3-methylamino-phenylthio)-estr-9(11)-en-5α,17β-diol (5B)

9,4 g 5A und 2,5 g Lithiumaluminiumhydrid werden in 400 ml THF abs. bei 80 °C unter Argon 1h am Rückfluß erhitzt.

Anschließend wird überschüssiges Lithiumaluminiumhydrid mit Wasser hydrolysiert, mit Essigester verdünnt, die org. Phase abgetrennt und mit Wasser gewaschen, über $Na_2SO_4$ getrocknet und das Lösungsmittel am Rotationsverdampfer abgezogen.
Ausbeute nach Säulenchromatographie: 8,4 g 5B, Schaum

**C.** 3,3-(2,2-Dimethyltrimethylendioxy)-10β-(3-N-formyl-N-methylaminophenylthio)-estr-9(11)-en-5α,17β-diol (5C)

Durchführung und Aufarbeitung wie Beispiel 5A.

Eingesetzte Mengen: 8,6 g 5B, 3,39 ml Acetanhydrid, 1,4 ml HCOOH, 45 ml Pyridin p.a.
Ausbeute nach Säulenchromatographie: 6,0 g 5C, Schaum

**D.** 10β-(3-Dimethylaminophenylthio)-3,3-(2,2-dimethyltrimethylendioxy)-estr-9(11)-en-5α,17β-diol (5D)

3,95 g 5C werden in 150 ml abs. THF gelöst und bei 0 °C unter Argon mit 1,46 ml einer 10 molaren Borandimethylsulfidkomplex-Lösung in THF versetzt. Es wird 2,5 h bei Raumtemperatur nachgerührt. Das Reaktionsgemisch wird bei 0 °C langsam mit 4 ml Methanol versetzt und über Nacht an der Luft stehengelassen. Anschließend wird mit $H_2O$ und Essigester verdünnt. Die organische Phase wird abgetrennt, mit $H_2O$ gewaschen, über $Na_2SO_4$ getrocknet und im Vakuum eingeengt.
Ausbeute nach Säulenchromatographie: 3,6 g 5D, Schaum

**E.** 10β-(2-Brom-5-dimethylaminophenylthio)-3,3-(2,2-dimethyltrimethylendioxy)-estr-9(11)-en-5α,17β-diol (5E)

5 g 5D werden in 200 ml $CCl_4$ gelöst, bei 0 °C mit 1,65 g N-Bromsuccinimid versetzt und bei 0 °C unter Argon 2h gerührt. Das Reaktionsgemisch wird mit $CH_2Cl_2$ verdünnt und mit gesättigter $NaHCO_3$-Lösung versetzt, die org. Phase mit Wasser gewaschen und über $Na_2SO_4$ getrocknet und das Lösungsmittel am

Rotationsverdampfer abgezogen.
Ausbeute nach Säulenchromatographie: 2,7 g 5E, Schaum

**F.** 10β,11β-(5-Dimethylamino-o-phenylenthio)-3,3-(2,2-dimethyltrimethylendioxy)-estran-5α,17β-diol (5F)
Durchführung und Aufarbeitung wie bei Beispiel 1B.
Eingesetzte Mengen: 1,7 g 5E, 1,15 ml Tri-n-butylzinnhydrid, 30 mg Azobisisobutyronitril, 113 ml Toluol p.a.
Ausbeute nach Säulenchromatographie: 675 mg 5F, Schaum

**G.** 10β,11β-(5-Dimethylamino-o-phenylenthio)-3,3-(2,2-dimethyltrimethylendioxy)-5α-hydroxy-estran-17-on (5G)
Durchführung und Aufarbeitung wie bei Beispiel 2A.
Eingesetzte Mengen: 663 mg 5F, 150 mg Aluminiumtriisopropylat, 0,578 ml Cyclohexanon, 31 ml Toluol p.a.
Ausbeute nach Säulenchromatographie: 392 mg 5G, Schaum

**H.**
10β,11β-(5-Dimethylamino-o-phenylenthio)-3,3-(2,2-dimethyltrimethylendioxy)-17α-(1-propinyl)-estran-5α,17β-diol (5H)
Durchführung und Aufarbeitung wie bei Beispiel 2B.
Eingesetzte Mengen: 15 ml THF abs., 1h Propin-Strom, 2,8 ml n-Butyllithium (1,6M in Hexan), 390 mg 5G in 10 ml abs. THF.
Ausbeute nach Säulenchromatographie: 350 mg 5H

**I.** 10β,11β-(5-Dimethylamino-o-phenylenthio)-17β-hydroxy-17α-(1-propinyl)-estr-4-en-3-on (5I)
Durchführung und Aufarbeitung wie bei Beispiel 2C.
Eingesetzte Mengen: 340 mg 5H, 11 ml Aceton p.a., 0,54 ml 4N HCl.
Ausbeute nach Säulenchromatographie: 185 mg 5I, Schaum
$^1$H-NMR-Spektrum ($CD_2Cl_2$, 300 MHZ); δ (in ppm) 0,40 (s, H-18), 1,90 (s, H-22), 3,40 (ddbr, H-11), 5,85 (s, H-4), 2,88 (s, N-$CH_3$), 7,31 (d, 10Hz, H-3′) 6,53 (dd, 10,2Hz, H-4′), 6,38 (d, 2Hz, H-6′)

**Beispiel 6**

Darstellung von
10β,11β-(5-N,N-Dimethylamino-o-phenylenthio)-17β-hydroxy-17α-(3-hydroxyprop-1(Z)-enyl)-estr-4-en-3-on (6C)

A. 10β,11β-(5-N,N-Dimethylamino-o-phenylenthio)-3,3-(2,2-dimethyltrimethylendioxy)-17α-[3-tetrahydropyran-2-yloxy)-1-propinyl]-estran-5α,17β-diol (6A)
3,44 ml Propargyl-THP-ether werden in 120 ml absolutem Tetrahydrofuran vorgelegt und bei 0°C unter Argon mit 14, 4 ml n-Butyllithium (1,6 M in Hexan) versetzt. Nach Beendigung der Zugabe rührt man 30 Minuten bei 0°C, fügt 1,23 g 5G in 30 ml abs. Tetrahydrofuran zu und rührt anschließend 16 Stunden bei Raumtemperatur nach. Das Reaktionsgemisch wird mit gesättigter $NH_4Cl$-Lösung versetzt und mit Essigester verdünnt. Die organische Phase wird abgetrennt, zuerst mit gesättigter NaCl-Lösung, anschließend mit Wasser gewaschen, über Natriumsulfat getrocknet und im Vakuum eingeengt.
Ausbeute nach Säulenchromatographie: 890 mg 6A als Schaum.

B. 10β,11β-(5-N,N-Dimethylamino-o-phenylenthio)-(-3,3-(2,2-dimethyltrimethylendioxy)-17α-[3-(tetrahydropyran-2-yloxy)-prop-1(Z)-enyl]-estran-5α,17β-diol (6B)
850 mg 6A werden in 8,5 ml Ethanol p.A. und 8,5 ml Pyridin p.A. vorgelegt und unter Argon mit 85 mg Palladium auf Bariumsulfat als Katalysator versetzt. Anschließend wird 16 Stunden bei Raumtemperatur unter mechanischem Schütteln hydriert. Nach beendeter Wasserstoffaufnahme wird der Katalysator über Celite abgesaugt und das Filtrat im Vakuum eingeengt.
Ausbeute nach Säulenchromatographie: 650 mg 6B als Schaum.

C. 10β,11β-(5-N,N-Dimethylamino-o-phenylthio)-17β-hydroxy-17α-(3-hydroxyprop-1(Z)-enyl)-estr-4-en-3-on (6C)
600 mg 6B werden in 25 ml Aceton p.A. gelöst, mit 1,4 ml 4N HCl versetzt und 15 Minuten bei 60°C gerührt. Die Reaktionslösung wird mit gesättigter $NaHCO_3$-Lösung versetzt und mit Essigester extrahiert. Die organische Phase wird mit Wasser gewaschen, über $Na_2SO_4$ getrocknet und im Vakuum eingeengt.
Ausbeute nach Säulenchromatographie: 255 mg 6C als Schaum.
$^1$H-NMR-Spektrum ($CD_2Cl_2$, 300 MHz): δ (in ppm) 0,49 (s,H-18); 2,9 (s,$CH_3$-N), 3,36 (ddbr,H-11); 4,28 (m,H-22, H-21); 5,68 (m, H-20, H-21); 5,85 (s,H-4), 6,48 (d, 2Hz,H-6′); 6,52 (dd 10,2 Hz, H-4′); 7,3 (d, 10Hz, H-3′).

**Beispiel 7**

Darstellung von 10β,11β-(5-Acetyl-o-phenylenthio)-17β-hydroxy-17α-(3-hydroxy-prop-1(Z)-enyl)-estr-4-en-3-on (7C)

A. 3,3-(2,2-Dimethyltrimethylendioxy)-10β,11β-{5-[1,1-(2,2-dimethyltrimethylendioxy)-ethyl]-o-phenylenthio}-17α-[3-(tetrahydropyran-2-yloxy)-1-propinyl]-estr-5-en-17β-ol (7A)

Durchführung und Aufarbeitung wie bei Beispiel 6A.

Eingesetzte Mengen: 2,5 ml Propargyl-THP-ether in 85 ml absolutem Tetrahydrofuran, 10,5 ml n-Butyllithium (1,6 M in Hexan), 1,0 g 4F in 17,5 ml absolutem Tetrahydrofuran.

Ausbeute nach Säulenchromatographie: 1,08 g 7A, Schmelzpunkt: 156-157° C.

B. 10β,11β-(5-Acetyl-o-phenylenthio)-17β-hydroxy-17α-(3-hydroxy-1-propinyl)-estr-4-en-3-on (7B)

760 mg 7A werden in 26 ml Aceton p.A. gelöst, mit 1,56 ml 4N HCl versetzt und 40 Minuten bei Raumtemperatur sowie weitere 10 Minuten bei 60° C gerührt. Aufarbeitung wie bei Beispiel 6C.

Ausbeute nach Säulenchromatographie: 341 mg 7B als Schaum.

C. 10β,11β-(5-Acetyl-o-phenylenthio)-17β-hydroxy-17α-(3-hydroxyprop-1(Z)-enyl)-estr-4-en-3-on (7C)

Durchführung und Aufarbeitung wie bei Beispiel B.

Eingesetzte Mengen: 340 mg 7B gelöst in 5,3 ml Ethanol p.A., 5,3 ml Pyridin p.A., 53 mg Palladium auf Bariumsulfat.

Ausbeute nach Säulenchromatographie: 148 mg 7C als Schaum.

$^1$H-NMR-Spektrum ($CD_2Dl_2$, 300 MHz : δ(in ppm)

0,4 (s, H-18); 2,55 (s, H-Ac); 3,47 (ddbr, H-11); 4,3 (m, H-22); 5,7 (m, H-20, H-21); 5,9 (S, H-4); 7,65 (m, H-3′, H-4′, H-6′).

Analog zu den Verbindungen der vorstehend beschriebenen Beispiele lassen sich u.a. auch folgende erfindungsgemäße Verbindungen herstellen und sind Gegenstand der vorliegenden Erfindung:

17β-Hydroxy-17α-(3-hydroxyprop-1(Z)-enyl)-10β,11β-(5-methoxy-o-phenylenthio)-estr-4-en-3-on
10β-11β-(5-Dimethylamino-o-phenylenthio)-17β-hydroxy-17α-methoxymethyl-estr-4-en-3-on
17α-Cyanomethyl-10β,11β-(5-dimethylamino-o-phenylenthio)-17β-hydroxy-estr-4-en-3-on
10β,11β-(5-Dimethylamino-o-phenylenthio)-17β-hydroxy-17α-(3-hydroxyprop-1(Z)-enyl)-estr-4-en-3-on
10β,11β-(5-Acetyl-o-phenylenthio)-17β-hydroxy-estr-4-en-3-on
10β,11β-(5-Acetyl-o-phenylenthio)-17β-hydroxy-17α-methoxymethyl-estr-4-en-3-on
10β,11β-(5-Acetyl-o-phenylenthio)-17α-cyanomethyl-17β-hydroxy-estr-4-en-3-on
10β,11β-(5-Acetyl-o-phenylenthio)-17β-hydroxy-17α-(3-hydroxyprop-1(Z)-enyl)-estr-4-en-3-on
10β,11β-(5-Isopropenyl-o-phenylenthio)-17β-hydroxy-17α-methoxymethyl-estr-4-en-3-on
17α-Cyanomethyl-10β,11β-(5-isopropenyl-o-phenylenthio)-1 7β-hydroxy-estr-4-en-3-on
10β,11β-(5-Isopropenyl-o-phenylenthio)-17β-hydroxy-17α-(3-hydroxyprop-1(Z)-enyl)-estr-4-en-3-on
10β,11β-(5-Ethinyl-o-phenylenthio)-17β-hydroxy-17α-(1-propinyl)-estr-4-en-3-on.

## Patentansprüche

1.) 10β,11β-überbrückte Steroide der allgemeinen Formel (I)

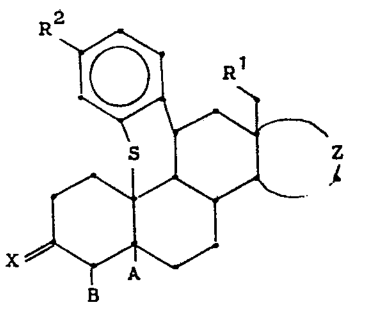

(I),

worin

$R^1$ für ein H-Atom oder eine Methylgruppe,

$R^2$ für ein H-Atom, einen Cyanidrest, einen Heteroarylrest, eine geradkettige oder verzweigte, gegebenenfalls Doppel- oder Dreifachbindung(en) aufweisende Alkylgruppe mit bis zu 20 C-Atomen, die gegebenenfalls durch eine oder mehrere Oxo-Gruppe(n) substituiert ist, eine $OR^3$-, $SR^3$-, $-OSO_2-R^{11}$-Gruppe mit $R^{11}$ in der Bedeutung einer perfluorierten $C_1$-$C_4$-Alkylgruppe oder $NR^3R^4$-Gruppe mit $R^3$ in der Bedeutung eines H-Atoms oder eines $C_1$-$C_8$-Alkylrestes, $R^4$ in der Bedeutung von $R^3$, eines Cyanid- oder eines $C_1$-$C_{10}$-Acylrestes oder $R^3$ und $R^4$ gemeinsam unter Einschluß von N in der Bedeutung eines 5- oder 6-gliedrigen heterocyclischen Rings, wobei im Ring noch ein weiteres Heteroatom N, O oder S enthalten sein kann,

A und B entweder gemeinsam für eine weitere Bindung zwischen $C_4$ und $C_5$ oder A für eine $\alpha$-Hydroxygruppe und B für ein H-Atom,
X für eine Keto- oder Oximgruppe und
Z für den Rest eines pentagonalen oder hexagonalen Ringes, der gegebenenfalls substituiert und gegebenenfalls ungesättigt ist,
stehen,
sowie gegebenenfalls deren pharmazeutisch verträgliche Additionssalze mit Säuren.

2.) Verbindungen nach Anspruch 1, dadurch gekennzeichnet, daß Z für den Rest eines Ringes der Formel

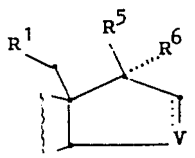

steht, worin
$R^1$ die in Formel 1 genannte Bedeutung hat,
die von V ausgehende gestrichelte Linie die etwaige Anwesenheit einer Doppelbindung symbolisiert,
V für einen $CH_2$-, CH-, $CH_2CH_2$- oder $CHCH_2$-Rest steht und
$R^5/R^6$ $-OR^7/-C\equiv C-U$
$-OR^7/-\underset{\underset{O}{\|}}{C}-CH_2-R^8$
$-\underset{\underset{O}{\|}}{C}-CH_2-R^8/-OR^7$
$-\underset{\underset{O}{\|}}{C}-CH_2-R^8/-CH_3$
$-\underset{\underset{O}{\|}}{C}-CH_2-R^8/-H$
$-OR^7/-(CH_2)_m-CH_2-R^9$
$-OR^7/-CH=CH(CH_2)_k-CH_2-R^9$
$-OR^{10}/-H$
$-OR^{10}/-(CH_2)_k-C\equiv C-E$,
wobei E Halogen ist,

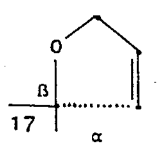

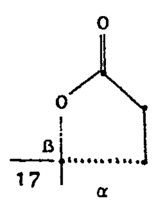

mit $R^7$ in der Bedeutung eines Wasserstoffatoms oder Acylrestes mit 1 bis 4 Kohlenstoffatomen, U in der Bedeutung eines Wasserstoffatoms, einer Alkyl-, Hydroxyalkyl-, Alkoxyalkyl- oder Acyloxyalkylgruppe mit jeweils 1 bis 4 Kohlenstoffatomen im Alkyl- bzw. Acylrest,
$R^8$ in der Bedeutung eines Wasserstoffatoms, einer Hydroxygruppe, einer Alkyl-, O-Alkyl- oder O-Acylgruppe mit jeweils 1 bis 4 Kohlenstoffatomen,
$R^9$ in der Bedeutung eines Wasserstoffatoms, eines Hydroxy- oder Cyanidrestes, einer O-Alkyl- oder O-Acylgruppe mit jeweils 1 bis 4 Kohlenstoffatomen,
$R^{10}$ in der Bedeutung eines Wasserstoffatoms, einer Alkyl- oder Acylgruppe mit jeweils 1 bis 10 Kohlenstoffatomen,
m in der Bedeutung 0, 1, 2 oder 3,
k in der Bedeutung 0, 1 oder 2,
bedeuten.

3.) 17β-Hydroxy-10β,11β-(5-methoxy-o-phenylenthio)-estr-4-en-3-on
17β-Hydroxy-10β,11β-(5-methoxy-o-phenylenthio)-17α-(1-propinyl)-estr-4-en-3-on
17β-Hydroxy-17α-(3-hydroxyprop-1(Z)-enyl)-10β,11β-(5-methoxy-o-phenylenthio)-estr-4-en-3-on
10β,11β-(5-Diethylamino-o-phenylenthio)-17β-hydroxy-17α-(1-propinyl)-estr-4-en-3-on
10β,11β-(5-Dimethylamino-o-phenylenthio)-17β-hydroxy-17α-(1-propinyl)-estr-4-en-3-on
10β,11β-(5-Dimethylamino-o-phenylenthio)-17β-hydroxy-17α-methoxymethyl-estr-4-en-3-on
17α-Cyanomethyl-10β,11β-(5-dimethylamino-o-phenylenthio)-17β-hydroxy-estr-4-en-3-on
10β,11β-(5-Dimethylamino-o-phenylenthio)-17β-hydroxy-17α-(3-hydroxyprop-1(Z)-enyl)-estr-4-en-3-on
10β,11β-(5-Acetyl-o-phenylenthio)-17β-hydroxy-estr-4-en-3-on
10β,11β-(5-Acetyl-o-phenylenthio)-17β-hydroxy-17α-(1-propinyl)-estr-4-en-3-on

10β,11β-(5-Acetyl-o-phenylenthio)-17β-hydroxy-17α-methoxymethyl-estr-4-en-3-on
10β,11β-(5-Acetyl-o-phenylenthio)-17α-cyanomethyl-17β-hydroxy-estr-4-en-3-on
10β,11β-(5-Acetyl-o-phenylenthio)-17β-hydroxy-17α-(3-hydroxyprop-1-(Z)-enyl)-estr-4-en-3-on
10β,11β-(5-Isopropenyl-o-phenylenthio)-17β-hydroxy-17α-methoxymethyl-estr-4-en-3-on
17α-Cyanomethyl-10β,11β-(5-isopropenyl-o-phenylenthio)-1 7β-hydroxy-estr-4-en-3-on
10β,11β-(5-Isopropenyl-o-phenylenthio)-17β-hydroxy-17α-(3-hydroxyprop-1( Z)-enyl)estr-4-en-3-on
10β,11β-(5-Ethinyl-o-phenylenthio)-17β-hydroxy-17α-(1-propinyl)-estr-4-en-3-on.

4.) Verbindungen der allgemeinen Formel II

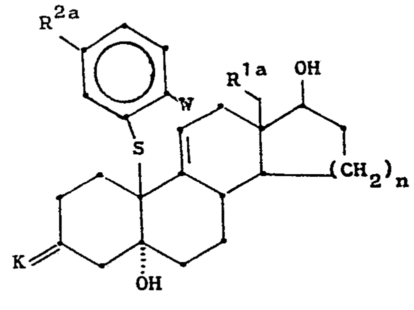

(II),

worin
$R^{1a}$ und $R^{2a}$ die gleiche Bedeutung haben wie $R^1$ bzw. $R^2$ in Formel I, wobei gegebenenfalls in $R^{2a}$ vorhandene Hydroxy-, Mercapto-, Amino , Oxo-und/oder terminale Acetylengruppen geschützt sind,
n für die Ziffern 1 oder 2,
K für eine in Form des Ketals oder Thioketals blockierte Ketogruppe
W für ein Brom- oder Jod-Atom stehen.

5.) 10β,11β-überbrückte Steroide der allgemeinen Formel III

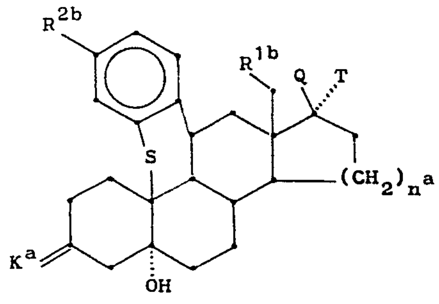

(III),

worin
$R^{1b}$, $R^{2b}$, $K^a$ und $n^a$ die gleiche Bedeutung haben wie $R^1$, $R^2$, K bzw. n, wobei jedoch gegebenenfalls eine in $R^{2b}$ vorhandene Hydroxy-, Mercapto-, Amino-, Oxo- und/oder terminale Acetylengruppen geschützt ist, und Q für $R^5$ und T für $R^6$ oder aber Q und T gemeinsam für ein Ketosauerstoffatom stehen.

6.) Verfahren zur Herstellung von Verbindungen der allgemeinen Formel I

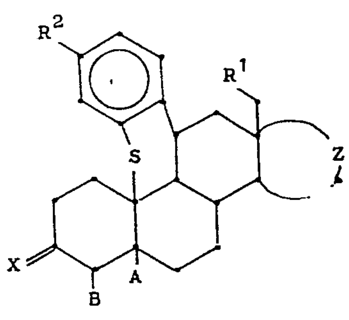

(I),

worin
die Substituenten $R^1$, $R^2$, X, A, B und Z die in Anspruch 1 angegebene Bedeutung haben,
dadurch gekennzeichnet, daß man Verbindungen der allgemeinen Formel II

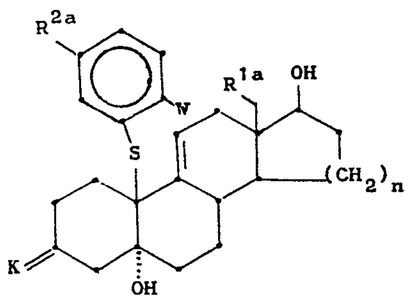

(II),

worin
$R^{1a}$ und $R^{2a}$ die gleiche Bedeutung wie $R^1$ bzw. $R^2$ in Formel I, ausgenommen $OSO_2$-$R^{11}$, sowie n, K und W die in Formel I angegebene Bedeutung haben, wobei gegebenenfalls vorhandene Hydroxy-, Mercapto-, Amino-, Oxo- und/oder terminale Acetylengruppen geschützt sind,
zu den Zwischenprodukten der allgemeinen Formel IIIa cyclisiert

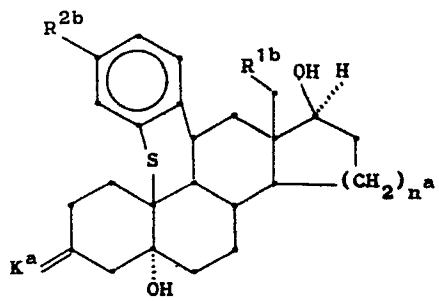

(IIIa),

worin
$R^{1b}$, $R^{2b}$, $K^a$ und $n^a$ die in Formel II angegebene Bedeutung haben
und dann entweder zuerst
a) die C-17-Hydroxygruppe gegebenenfalls oxidiert und anschließend
b) gegebenenfalls eine eine Schutzgruppe aufweisende Hydroxygruppe am Phenylenring von dieser Schutzgruppe befreit, gewünschtenfalls aus der Hydroxyverbindung ein entsprechendes Perfluoralkylsulfonat herstellt, gegebenenfalls das Perfluoralkylsulfonat mit einer entsprechend substituierten Zinntrialkylverbindung in eine Verbindung überführt, die am Phenylenring, gegebenenfalls nach weiteren Reaktionen, das gewünschte Substitutionsmuster aufweist,
oder zuerst b) und dann a) ausführt und danach gegebenenfalls
c) den Ring D in gewünschter Weise nach an sich bekannten Methoden funktionalisiert, das so erhaltene Produkt der Einwirkung eines Dehydratationsmittels, das auch zur Freisetzung der 3-Oxogruppe und gegebenenfalls weiterer geschützter Gruppen befähigt ist, unter Ausbildung der 4(5)-Doppelbindung unterwirft
oder
d) das so erhaltene Produkt der Einwirkung eines Dehydratationsmittels, das auch zur Freisetzung der 3-Oxogruppe und gegebenenfalls weiterer geschützter Gruppen befähigt ist, unter Ausbildung der 4(5)-Doppelbindung unterwirft und anschließend, nach Schutz der 3-Oxogruppe, den Ring D in gewünschter Weise funktionalisiert,
oder die Schritte a) und b) oder b) und a) nach dem Schritt c) oder d) ausführt,
das so erhaltene Produkt gegebenenfalls von Schutzgruppen befreit, gewünschtenfalls die am Phenylenring gegebenenfalls vorhandene Hydroxy-, Mercapto- und/oder Aminogruppe alkyliert bzw. acyliert, gewünschtenfalls einen Cyanidrest in den Phenylenring einführt, gewünschtenfalls mit Hydroxylamin-hydrochlorid zum Produkt der allgemeinen Formel I mit X in der Bedeutung der Hydroxyiminogruppierung N~OH umsetzt sowie gegebenenfalls ein pharmazeutisch verträgliches Säureadditionssalz herstellt.

7.) Verfahren nach Anspruch 6, dadurch gekennzeichnet, daß die Verbindungen der allgemeinen Formel II unter reduktiv radikalischen Bedingungen zu den Zwischenprodukten der allgemeinen Formel IIIa cyclisiert werden.

8.) Verfahren nach Anspruch 7, dadurch gekennzeichnet, daß die Verbindungen der allgemeinen Formel II mit Tributylzinnhydrid in Gegenwart von Azobisisobutyronitril cyclisiert werden.

9.) Verfahren nach einem der Ansprüche 6-8, dadurch gekennzeichnet, daß das Perfluoralkylsulfonat mit einer entsprechend substituierten Zinntrialkylverbindung unter Zusatz eines Katalysators in eine Verbindung überführt wird, die am Phenylenring, gegebenenfalls nach weiteren Reaktionen, das gewünschte Substitutionsmuster aufweist.

10.)Verfahren nach Anspruch 9, dadurch gekennzeichnet, daß als Katalysator eine Übergangsmetallverbindung, vorzugsweise eine Pd°-Verbindung, verwendet wird.

11.)Pharmazeutische Präparate, gekennzeichnet durch einen Gehalt an Verbindungen gemäß den Ansprüchen 1 bis 5.

12.)Verwendung von Verbindungen gemäß den Ansprüchen 1 bis 5 zur Herstellung von Arzneimitteln.